Europäisches Patentamt

European Patent Office

Office européen des brevets

(11) Publication number : **0 641 562 A1**

## (12) EUROPEAN PATENT APPLICATION

(21) Application number : 94810514.3

(22) Date of filing : 06.09.94

(51) Int. Cl.$^6$ : **A61K 31/20,** A61K 31/195, A61K 31/70, A23L 1/305, A23L 1/30

(30) Priority : 08.09.93 GB 9318611

(43) Date of publication of application :
08.03.95 Bulletin 95/10

(84) Designated Contracting States :
AT BE CH DE DK ES FR GB GR IE IT LI LU NL PT SE

(71) Applicant : SANDOZ NUTRITION LTD.
Monbijoustrasse 118
CH-3001 Berne (CH)

(72) Inventor : Schneider, Heinz
Megglete 3
CH-1792 Cordast (CH)

(74) Representative : Smolders, Walter et al
Sandoz Technology LTD.,
Patents and Trademarks Division,
Lichtstrasse 35
CH-4002 Basle (CH)

(54) Nutritional supplement.

(57) Method of stimulating the immune system of patients suffering from HIV-related infection, by administering a nutritional supplement and the use of omega-3 PUFA and a compound associated with the synthesis of polyamines in the manufacture of such supplement.

EP 0 641 562 A1

This invention relates to a method of stimulating the immune system of patients suffering from human immunodeficiency virus (HIV)-related infection, by administering a nutritional supplement and to the use of a specific components in the manufacture of the nutritional supplement.

HIV-infection is frequently accompanied by malnutrition (see Malnutrition and Immune Dysfunction in Patients Infected with HIV, by U. Süttmann et al. in Klin. Wochenschr. (1991) 69: 156-162). Although HIV-related malnutrition is regarded to be one of the earliest clinical signs of the disease, it is generally accepted that there is no direct correlation between immunological depression and the loss of body mass.

As long as it is not possible to cure HIV-infected patients, it is important to slow down or reverse the development of adverse symptoms associated with HIV-infection whilst allowing the patients to continue as long as possible their normal daily life, including eating habits. From the nutritional point of view there is accordingly a need to develop a dietary supplement having immunostimulatory activity and that can be taken between meals. Thus there is a need for a dietary supplement that stimulates the immune system when administered in relatively low amounts, the immunostimulatory activity of the supplement not being impaired by previous or subsequent meals taken by the HIV-infected patient.

The present invention relates to the use of a compound associated with the synthesis of polyamines(which hereinafter will be referred to as component (a)); and an omega-3-polyunsaturated fatty acid (which hereinafter will be referred to as component (b)) in the manufacture of a nutritional supplement for treatment of patients suffering from Human Immunodeficiency Virus-related infection.

The present invention further relates to the use of component (a); component (b) and a nucleobase source in the manufacture of a nutritional supplement for treatment of patients suffering from human immunodeficiency virus-related infection.

In a further embodiment the present invention provides a method of stimulating the immune system in patients suffering from Human Immunodeficiency Virus-related infection which method comprises administering to the patient an aggregate immunostimulatory effective amount of component (a) and component (b).

In a yet further embodiment the present invention provides a method of stimulating the immune system in patients suffering from Human Immunodeficiency Virus-related infection which method comprises administering an aggregate immunostimulatory effective amount of component (a), component (b) and optionally a nucleobase source.

For convenience, the mixture comprising components (a), (b) and optionally a nucleobase source is designated hereinafter Supplement of the Invention.

The Supplements of the Invention surprisingly stimulate the immune system of HIV-patients after administration in low amounts. Typical effects observed after intake of the Supplements of the Invention are an increase of the serum concentrations of both TNF receptors and/or a significant increase of the CD4 helper cells concentration. The immunostimulatory activity of the supplement is not impaired by meals taken by the patients. The Supplements of the Invention are particularly suitable for administration to patients suffering from HIV-infection in the stages WR-1 to WR-4 of the Walter Reed disease stage classification. Particularly surprising is that the Supplements of the Invention allow a significant weight gain and that a substantial proportion of the weight gain is due to increase of lean body mass rather than fat.

The term "compound associated with the synthesis of polyamines" as used herein is intended to include, but not be limited to arginine, arginine precursors, ornithine and the like; it refers in particular to arginine and ornithine; preferably to arginine. Amino acids having a bitter taste, including arginine and ornithine, are conveniently employed in low pH form, i.e. in a form having when dissolved in water, a pH of less than 7.

For determination of the pH, the concentration of the amino acid form in water is not very critical and can vary within very wide ranges as will be appreciated by the skilled person; appropriate concentrations lie in the range of from ca. 0.1 % by weight to saturation of the solution to ca. 20 % by weight resp. Any low pH form, such as a salt or ionic species of the amino acid, is useful in this invention, provided that it is physiologically acceptable and does not have an objectionable taste. Low pH forms include acid addition salts of food grade acids such as phosphoric, citric, adipic, tartaric, acetic, fumaric, malic and lactic acid and the like and mixtures thereof. Where the acid is di- or tri-basic, the low pH salt form may be a di- or tri-salt, e.g. a di- or tri-phosphate. The low pH amino acid forms also include ionic species having an acidic aqueous pH of less than 7 or formed in situ at acidic pH. Examples of suitable ionic species include, but are not limited to, the cations of the amine acid salts listed above. The salts and ionic species may be in hydrate form.

The low pH form of the amino acids of this invention, such as arginine phosphate for example, can be prepared by dissolving the arginine (free base) in water at a concentration slightly under saturation (i.e. 15 % w/w).

For the purpose of the invention, the omega-3 polyunsaturated fatty acids (PUFA) may be in free acid form or in a form suitable for the physiological supply of omega-3 PUFAs, e.g. in triglyceride form. Examples of omega-3 PUFAs, particularly appropriate for use in the compositions of the invention, include eicosapentaenoic acid (EPA ; 20:5 n-3), docosahexaenoic acid (DHA; 22:6 n-3) and $\alpha$-linolenic acid (LNA; 18:3 n-3). Suitable sources for omega-3 PUFAs include omega-3 PUFAs of vegetable source and fish oils, such as linseed oil and fish oils such as menhaden oil, salmon oil, mackeral oil, tuna oil and anchovy oil. The omega-3 PUFAs may be in free form or in microencapsulated form. Microencapsulated omega-3 PUFAs are known in the art and commercially available. They may be obtained in a manner known per se.

Nucleobase sources suitable for use according to the invention comprise or consist of natural nucleobases, nucleosides, nucleotides, RNA, DNA, equivalents thereof and/or mixtures comprising one or more of these. Nucleobase sources are added as an optional component, primarily as a nucleoside precursor.

Natural nucleobases include the purines, adenine and guanine, as well as the pyrimidines cytosine, thymine and uracil. Where the nucleobase source is in the form of free nucleobases, it is preferably uracil.

Natural nucleosides include the ribose nucleosides adenosine, guanosine, uridine and cytidine and the deoxyribose nucleosides deoxyadenosine, deoxyguanosine, deoxythymidine and deoxycytidine.

Natural nucleotides include phosphate esters of natural nucleosides, such as the monophosphates adenylate (AMP), guanylate (GMP), uridylate (UMP), cytidylate (CMP), deoxythymidylate (dTMP) deoxycytidylate (dCMP), and diphosphates and triphosphates of natural nucleosides such as ADP and ATP.

A purified nucleobase source, such as yeast, is preferred.

For the purpose of this invention, one unit weight of nucleobase is regarded as being equivalent to 2.5 to 3.0 unit weight of RNA, DNA, nucleosides or nucleotides.

Where the supplement comprises a nucleobase source in the form of a free nucleobase , the nucleobase is present in the range of 0.03 to 1.6 g. Where the nucleobase source is a nucleoside,a nucleotide, RNA, DNA or a mixture of one or more thereof, the nucleobase source is present in the range of from 0.1 to 4.0 g. Thus in the range of 0.1 to 4.0g RNA or an equivalent amount of another nucleobase source may be present.

The supplement of the invention conveniently comprises from 1 to 50 g component (a) and from 0.1 to 20 g component (b) in formulations supplying from 300 to 2000 Kcal/day. Preferably the supplement comprises from 0.2 to 10 g component (b) and most preferably from 0.6 to 5.0 g component(b) in formulations supplying from 300 to 2000 Kcal/day.

Preferably the supplement is formulated so that the recommended daily intake of components a) and b) supplies from 300 to 1200 Kcal./day, more preferably from 300 to 900 Kcal/day and most particularly from 300 to 700 Kcal./day. The recommended daily intake of supplement will depend on a number of factors, in particular the size and weight of the patient together with the average daily calorie intake obtained from ingestion of energy sources in addition to the supplement.

The Supplements of the Invention are particularly suitable for oral ingestion, but may also be administered to the gastro-intestinal tract via a feeding tube. The Supplements of the Invention are preferably in aqueous form or in a dry powder form, whereby the powder is conveniently added to water prior to use. Where the patient is taking the supplement orally, the supplement can be taken once daily. It is however preferred that the supplement be taken in multiple dose form e.g two to four times daily, more preferably two times daily. Preferably each dose should not supply more than 300 Kcal., e.g. from 75 to 300 Kcal. The Supplement of the Invention may be supplied in powder form or as a solution. Where the supplement is in powder form it may be mixed with water and taken as a drink between meals. Typical examples of Supplements of the Invention supply from 3.0 to 20.0 g of component (a), from 0.6 to 5.0 g of component (b) and from 300-700 Kcal/day.

Examples of energy sources suitable for incorporation in the Supplement of the Invention include nutritionally acceptable nitrogen sources, carbohydrate sources and fat sources.

Examples of suitable nitrogen sources for the Supplements of this Invention, include nutritionally acceptable proteins such as whey proteins, caseinates and protein hydrolysates. Suitable carbohydrate sources include for example dextrins such as maltodextrins, sucrose, glucose, fructose and starch. In these forms the carbohydrates are both energy suppliers and sweeteners. The lactose content is conveniently kept low, it is preferably below 1 % by weight, more preferably below 0.2 % by weight of the carbohydrates. Suitable fatty acid sources include for example omega-6 PUFA sources having a high linoleic acid content such as safflower oil, sunflower oil, soya oil, cotton oil and corn oil and triglycerides supplying omega-3 PUFAs, omega-6 PUFAs, mono-unsaturated omega-9 fatty acids, medium chain fatty acids (i.e. $C_6$ to $C_{12}$ fatty acids) or mixtures thereof.

According to another embodiment the present invention provides a method of stimulating the immune system in patients suffering from Human Immunodeficiency Virus-related infection comprising administering to the patient an aggregate immunostimulatory effective amount of component (a), component (b), optionally a nucleobase source and vitamin C, E or A or a mixture of one or more of said vitamins.

Thus the Supplements of the Invention preferably comprises an amount of the Vitamin C and E, and - where desired - Vitamin A, sufficient to avoid re-oxidation of component (b). Higher amounts of vitamins may be added where this is indicated, in view of the particular needs of the patient and insofar as the amounts are physiologically acceptable. Examples of vitamins suitable for incorporation in the composition of the invention include Vitamin A, Vitamin D, Vitamin E, Vitamin K, Vitamin C, folic acid, thiamin, riboflavin, Vitamin $B_6$, Vitamin $B_{12}$,

niacin, biotin and panthotenic acid in pharmaceutically acceptable form.

The Supplements of the Invention may optionally comprise further vitamins in addition to those mentioned above, minerals, trace elements, flavorings, colorants, sweeteners and a variety of other ingredients useful in nutritional compositions.

Sweeteners can include any of the natural or artificial sweetening agents known in the art, but preferably the sweetener is sucrose, fructose, maltodextrin or aspartamine, or a mixture thereof.

Preferred flavorings for the compositions are the natural and artificial acid flavorings such as lemon, lime, orange, peach, pineapple and raspberry and the like.

If component (a) is in low pH form, the Supplements of the invention conveniently have a pH ranging from about 3 to about 7, more preferably from about 3 to about 6, most preferably from about 3 to about 5. Accordingly any protein comprised in the Supplements of the Invention should conveniently be stable and not coagulate over a wide pH range, e.g. of about pH 3-7.

Examples of mineral elements and trace elements suitable for incorporation in the composition of the invention include sodium, potassium, calcium, phosphorous, magnesium, manganese, copper, zinc, iron, selenium, chromium and molybdenum in pharmaceutically acceptable form.

The following examples are presented to further illustrate this invention. The examples are intended in an illustrative sense and not a limitative sense. The invention includes the embodiments shown and described herein and equivalents thereof.

## EXAMPLE 1

The effect of two nutritional supplements on HIV-infected patients is compared, a standard supplement(S) and a Supplement of the Invention(A).

The Standard supplement (Formulation S - see Table 1 below) is substantially free from $\alpha$- linolenic acid, arginine and RNA. The Supplement of the Invention (Formulation A; - see Table 1 below) is based on the Standard supplement but enriched with 2.75 g/1000 ml of $\alpha$-linolenic acid, 12.4 g/1000 ml arginine and 1.2 g/1000 ml yeast RNA. The energy supply of Supplement A is adjusted such that both supplements supply the same amount of energy per volume unit.

### TABLE 1 -

| Compositions A and S (major components) | | Compositions | |
|---|---|---|---|
| | | A | S |
| Protein | g | 27.9 | 21.7 |
| - Caseinate protein | g | 21.7 | 21.7 |
| - L-arginine | g | 6.2 | - |
| Fat, including | g | 13.9 | 13.9 |
| - linoleic acid (an omega-6 FA) | g | 1.8 | 3.6 |
| - $\alpha$-linolenic acid (an omega-3 FA) | g | 1.4 | 0.07 |
| - $\gamma$-linolenic acid | g | 0.1 | - |
| Carbohydrates | g | 67.0 | 73.23 |
| Yeast RNA | g | 0.6 | - |
| Energy | Kcal | 504.7 | 504.7 |

Randomised patient group I is treated for 4 months with Formulation S and the following 4 months with Formulation A.

Randomised patient group II is treated for 4 months with Formulation A and the following 4 months with Formulation S.

The supplements are administered orally as a nutritional supplement in the form of a drink solution in a total daily amount of 500 ml formulation (supplying 504.7 Kcal).

Several parameters are determined including weight increase, increase of lean body mass, development of skin folds at the beginning of the clinical test and at the end of the 8 months treatment (i.e. without distinction between the patients initially treated with Formulation A and the patients initially treated with Formulation S).

The weight gain will of course depend on the period of treatment of the patient. Results given below are considered statistically significant if the p-value is less than 0.05. An average weight gain over a four month period of 2.85Kg has been demonstrated, the result having a p-value of < 0.05 and thus being statistically significant. The weight gain is primarily an increase of lean body mass as indicated by a significant increase of the upper arm circumference and substantially unchanged skinfolds (subskin fat tissue).

It should be noted that the average weight gain in patents treated with Formulation S over a four month period is 0.54 Kg. Said average weight increase is however significally lower than that observed after treatment with Formulation A.

After 4 months treatment with Formulation A, the patients show an highly significant increase of the serum concentrations of both soluble TNF receptors (Type A [75 KD] and Type B [55 KD]). Thus over a four month period an increase of 0.9ng/ml soluble 55 KD TNF-receptor and an increase of 0.9 ng/ml soluble 75 KD TNF-receptor has been demonstrated; the p-value in each case being < 0.01. Such an increase is not observed after 4 months treatment with Formulation S, on the contrary a decrease in the level of soluble TNF-receptor may be observed.

In a second study the CD4 cells/$\mu$l increased after 12 weeks treatment of HIV patients with Supplement A from 319 to 431, i.e. by ca. 35 %; the p-value being 0.026. There was no significant increase of CD4 cell concentration after treatment with Formulation S.

## EXAMPLE 2

The following (Table 2) is an example of a suitable composition of the Supplement of the Invention in powder form.

## Supplement in Powder Form

| | | |
|---|---|---|
| Content | g | 152 |
| Protein | g | 33.5 |
| - caseinate protein | g | 26.0 |
| - L-arginine | g | 7.5 |
| | | |
| Fat | g | 16.8 |
| including | | |
| - omega-6 (FA) | g | 2.3 |
| - omega-3 (FA) | g | 1.7 |
| - gama linolenic acid | g | 0.12 |
| Carbohydrates | g | 80.4 |
| Fibre (ballast) | g | 10.0 |
| | | |
| Energy | kcal | 607 |
| | | |
| Na | mg | 800 |
| K | mg | 1000 |
| Ca | mg | 600 |
| Mg | mg | 200 |
| P | mg | 540 |
| Cl | mg | 1200 |
| Fe | mg | 9.0 |
| Cu | mg | 1.2 |
| Mn | mg | 1.5 |
| Zn | mg | 11.2 |
| F | mg | 1.2 |
| I | mcg | 112.0 |
| Cr | mcg | 75.0 |
| Mo | mcg | 150.0 |

| | | |
|---|---|---|
| Se | mcg | 35.0 |
| Retinol (Vit. .A.) | mg | 0.75 |
| Calciferol (Vit. D) | mcg | 5.00 |
| Tocopherol (Vit. E) | mg | 10.00 |
| Phylloquinone (Vit. K1) | mcg | 50.00 |
| Thiamin (Vit. B1) | mg | 0.90 |
| Riboflavin (Vit. B2) | mg | 1.30 |
| Pyridoxine (Vit. B6) | mg | 1.10 |
| Cyanocobalamin (Vit. B12) | mcg | 3.00 |
| Ascorbic acid (Vit. C) | mg | 50.00 |
| Biotin | mcg | 75.00 |
| Folic acid | mcg | 150.00 |
| Niacinamide | mg | 12.00 |
| Panthothenic acid | mg | 6.00 |
| Choline | mg | 200.00 |
| RNA | mg | 720.00 |

The above supplement may be mixed with water and taken in the appropriate concentration between meals.

## Claims

1. The use of a compound associated with the synthesis of polyamines (component a); and an omega-3 polyunsaturated fatty acid (component b) in the manufacture of a nutritional supplement for treatment of patients suffering from human immunodeficiency virus-related infection.

2. The use as claimed in Claim 1, wherein a nucleobase source is used in combination with the use of components (a) and (b).

3. The use as claimed in either Claim 1 or 2, wherein the nucleobase source is yeast RNA.

4. The use as claimed in any of the preceding Claims, wherein component (a) is arginine.

5. The use as claimed in any one of Claims 1 to 4, wherein component (b) is selected from the group consisting of eicosapentaenoic acid, decosahexanoic acid and $\alpha$- linolenic acid.

6. The use as claimed in Claim 5, wherein component (b) is in triglyceride form.

7. The use as claimed in any one of Claims 1 to 4, wherein component (b) is linseed oil and/or fish oil.

8. The use as claimed in any one of the preceding Claims, wherein the supplement comprises 1 to 50 g of component (a) and from 0.1 to 20 g of component (b), the supplement supplying from 300 to 2000 Kcal.

9. The use as claimed in Claim 8, wherein the supplement comprises from 0.2 to 10 g of component (b).

10. The use as claimed in Claim 9, wherein the supplement comprises from 0.6 to 5.0 g of component (b).

**11.** The use as claimed in any one of Claims 2 to 10, wherein the supplement comprises from 0.1 to 4.0 g of RNA or an equivalent amount of another nucleobase source

**12.** The use as claimed in any one of the preceding Claims, the supplement supplying from 300 to 2000 Kcal.

**13.** The use as claimed in Claim 12, the supplement supplying from 300 to 900 Kcal.

**14.** The use as claimed in Claim 13, the supplement supplying from 300 to 700 Kcal.

**15.** The use as claimed in any one of the preceding Claims, wherein the supplement comprises Vitamin C , E or A or a mixture of one or more thereof.

European Patent Office

# EUROPEAN SEARCH REPORT

Application Number

EP 94810514.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 6) |
|---|---|---|---|
| X | EP - A - 0 367 724<br>(SANDOZ AG)<br>* Abstract; claims 1-11,13;<br>page 2, line 3 - page 3,<br>line 58; example 1 * | 1-15 | A 61 K 31/20<br>A 61 K 31/195<br>A 61 K 31/70<br>A 23 L 1/305<br>A 23 L 1/30 |
| X | CH - A - 678 918<br>(BIOREX KFT)<br>* Abstract; claims; page 2,<br>line 3 - page 3, line 4;<br>example 13; page 4,<br>lines 47-57 * | 1,4,5,<br>8-10,<br>15 | |
| X | WO - A - 88/06 035<br>(SHRINERS HOSPITALS FOR<br>CRIPPLED CHILDREN)<br>* Abstract; claims 1-3,5;<br>page 8, line 19 - page 9,<br>line 8; page 20, lines 3-28 * | 1,4,<br>5-10,<br>12-15 | |
| A | US - A - 4 920 098<br>(R. COTTER et al.)<br>* Abstract; claims 1-3;<br>column 2, line 37 - column 3,<br>line 17; column 3, line 43 -<br>column 4, line 18 * | 1,4-7 | TECHNICAL FIELDS SEARCHED (Int. Cl. 6)<br><br>A 61 K 31/00<br>A 23 L 1/00 |
| A | EP - A - 0 524 796<br>(EFAMOL MOLDINGS PLC)<br>* Abstract; claims 1,2,6;<br>page 3, line 14 - page 4,<br>line 58; page 6, lines 1-28 * | 1,<br>6-10,<br>15 | |
| A | US - A - 5 221 668<br>(M.F. HENNINGFIELD et al.)<br>* Abstract; claims 1,2,10,<br>11,18-22; column 1, line 30 -<br>column 2, line 20;<br>column 2, lines 61-68 * | 1,4,5,<br>8-10,<br>12,15 | |

The present search report has been drawn up for all claims

| Place of search | Date of completion of the search | Examiner |
|---|---|---|
| VIENNA | 29-11-1994 | MAZZUCCO |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)